(19) Europäisches Patentamt / European Patent Office / Office européen des brevets

(11) **EP 2 093 685 A1**

(12) **EUROPEAN PATENT APPLICATION**

(43) Date of publication:
**26.08.2009 Bulletin 2009/35**

(51) Int Cl.:
***G06F 19/00*** *(2006.01)*     ***A61M 5/142*** *(2006.01)*

(21) Application number: **08003164.4**

(22) Date of filing: **21.02.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicants:
- **F.HOFFMANN-LA ROCHE AG**
  **4070 Basel (CH)**
  Designated Contracting States:
  **AT BE BG CH CY CZ DK EE ES FI FR GB GR HR HU IE IS IT LI LT LU LV MC MT NL NO PL PT RO SE SI SK TR**
- **Roche Diagnostics GmbH**
  **68305 Mannheim (DE)**
  Designated Contracting States:
  **DE**

(72) Inventors:
- **Remde, Axel**
  **3432 Lützelflüh-Goldbach (CH)**
- **Kalt, Lucas**
  **3014 Bern (CH)**

(74) Representative: **Poredda, Andreas et al**
**Disetronic Medical Systems AG**
**Kirchbergstrasse 190**
**3401 Burgdorf (CH)**

(54) **Drug administration device having a bolus administration profile controller**

(57)     Administration device for the subcutaneous administration of a liquid drug over an extended period of time, comprising:

a) an administration unit, the administration unit comprising a drug reservoir and a pump unit,
b) a housing, the housing comprising the administration unit, the housing being adapted to be carried by a patient over an extend period of time,
c) a controller unit,
characterized in that the controller unit further comprises
d) a bolus administration profile controller, the bolus administration profile controller being adapted to determine a bolus administration profile of a bolus to be administered, the bolus having a bolus volume B, wherein the bolus administration profile is determined, at least in part, based on the bolus volume B such that the depot volume B_depot of a depot is limited.

Figure 7

**Description**

The present invention is related to an administration device for the subcutaneous administration of a liquid drug over an extended period of time and to a method for controlling the subcutaneous administration of a bolus of a liquid drug by an administration device. In particular embodiments, the invention is related to administration devices for diabetes therapy using Continuous Subcutaneous Insulin Infusion (CSII) and to a method for the administration of insulin boli.

Continuous Subcutaneous Insulin Infusion (CSII) is the basis for a state-of-the-art therapy of insulin-dependent diabetes mellitus. In this therapy, a diabetic patient carries a miniaturized infusion pump virtually permanently night and day and preferably concealed from view. The infusion pump administers insulin via a cannula, the cannula being, e.g., made from medical-grade stainless steel or Teflon, into the subcutaneous fat tissue. The cannula may also be an arrangement of several cannulas, a micro needle array, or the like.

According to a first administration regime, the insulin pump administers insulin in a continuous or quasi-continuous way as defined by patient-specific and time-of-day dependent basal profile in order to cover the patient's basal (i.e., meal-independent) insulin need. According to a second administration regime, the pump is adapted to administer comparatively large insulin boli on demand which are required for covering the intake of food, namely carbohydrates, and to correct for undesirably high blood glucose values. Infusion devices which are adapted fort the CSII therapy of diabetes mellitus are disclosed, among others, in US 6878132.

While the (quasi-)continuous basal administration typically comprising small and virtually negligible drug volumes, bolus volumes are considerable in some cases, often resulting in bad and delayed drug absorption and high internal tissue pressure, potentially resulting in tissue damage. In some cases, the insulin may, in total or in part, flow back from the cannula tip along the outer cannula surface and leave the body, resulting in potentially dangerous lack of drug administration.

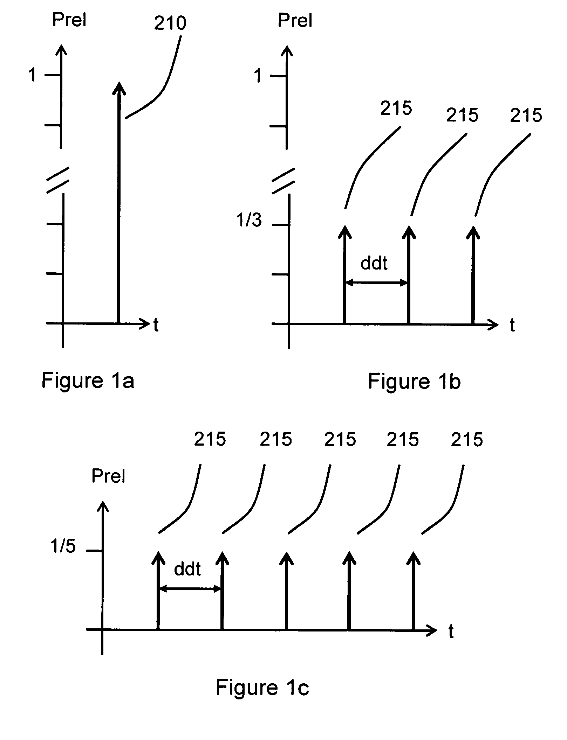

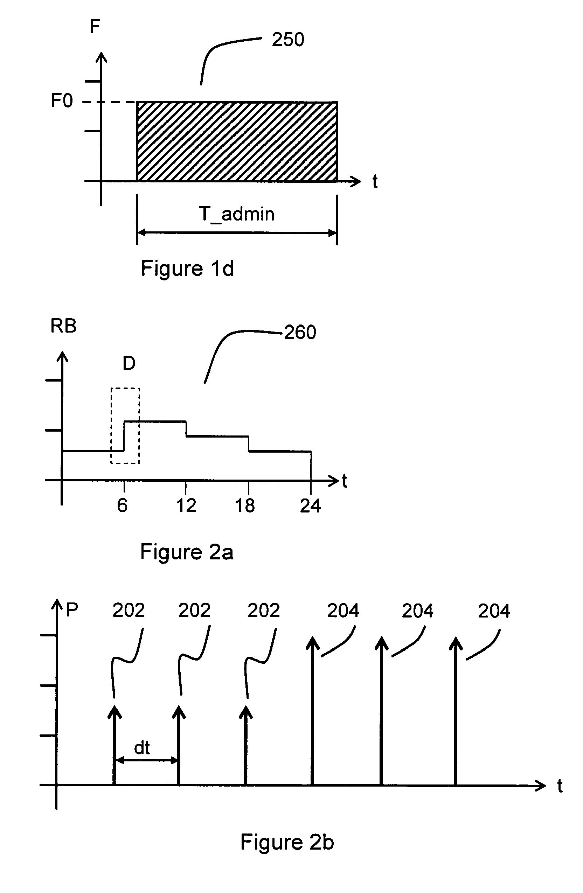

In the following, some terms related to subcutaneous drug administration are defined as they are used in the context of this invention. For illustration purposes, reference is made to Figure 1 and Figure 2 which do not limit the scope of the invention.

Figure 1a to Figure 1d illustrate alternative bolus administration profiles.

Figure 2a illustrates a typical basal profile over the time of day while Figure 2b illustrates a detail of an exemplary basal administration profile for basal administration according to Figure 2a.

The term 'absorption' refers to the uptake of the administered drug by the subcutaneous tissue. The term 'steady absorption' is used for an absorption while performing the drug administration while the term 'delayed absorption' is used for an delayed absorption from a depot as defined below. Absorption is generally dependent on the flow during administration and/or the average administration rate over an extended period of time, dependent on the administration regime. It is further dependent on factors such as the length and design of the administration cannula, the patient's tissue properties, and so on.

The term 'depot' refers to drug which is temporarily stored in the subcutaneous tissue prior to its delayed absorption by the tissue. In this context, a depot may be considered as a locally concentrated drug cloud in the tissue around the cannula.

The term 'flow' refers to the momentary flow, i.e. the drug volume which is administered per time, as a function of time, as it may, e.g., be measured by a flow sensor as a function of time t at the cannula. Flow is generally measured in volume per time, e.g., International Units per second, [IU/sec].

The term 'administration rate' refers to a more general measure for the drug volume which is administered per time and may be either an actual flow or an average flow over a given period of time. In a more general sense, the term 'rate' refers to a volume per time.

The term 'pulse' refers to Dirac administration pulses of negligible duration and defined drug volume. It must be understood, however, that the modeling of individual drug administrations as Dirac pulses is helpful for the mathematical and graphical representation in many cases, while the actual flow is generally complex depending on the design and the control of the administration device.

The term 'continuous' is used in the context of drug administration if the flow F is continuous and generally non-zero during the administration. Typically, but not necessarily, the flow F is substantially constant during the administration. In contrast, the term 'pulsed' is used if the flow F comprises pulses with the flow F being substantially zero between the pulses. It should be noted that an administration device may technically be operated in a pulsed way, using, e.g., a drive mechanism which is driven a stepper motor, while the flow F may be continuous due to fluid inertia and system elasticity.

The term 'bolus administration profile' refers to the distribution of a bolus volume B to be administered over time t. With the relative pulse volume P_rel being the pulse volume P of a pulse divided by the bolus volume B, the diagram shown in Figure 1a illustrates the administration of a bolus, the bolus having a bolus volume B, according to a first exemplary bolus administration profile, with the total bolus volume B being administered as a single immediate bolus pulse 210. The diagram shown in Figure 1b illustrates the administration of a bolus having the same bolus volume B according to a second exemplary bolus administration profile with the total bolus volume B being administered as three extended bolus pulses 215, each of the three extended bolus pulses 215 having a bolus pulse volume of 1/3*B, the bolus pulse intervals ddt between the extended bolus pulses 215 being equal. The diagram shown in Figure 1c illustrates the administration of a bolus having the same bolus volume B according to a third exemplary bolus administration profile with the total bolus volume B being administered as five extended bolus pulses 215, each of the five extended bolus pulses having a bolus pulse volume of 1/5*B, the bolus pulse intervals ddt between the extended bolus pulses 215 being equal. Figure 1d illustrates the administration of a bolus having the same bolus volume B according to a fourth exemplary bolus administration profile. According to this fourth bolus administration profile, the total bolus volume B is administered continuously with a substantially constant flow F0 over an administration time T_admin, such that the integral of the flow F over time t, i.e., the area F0*T_admin of rectangle 250, equals the bolus volume B. Similar further bolus administration profiles may be obtained, e.g., by performing the administration continuously with a substantially constant flow F1, the flow F1 being different from the flow F0, or by varying the flow F over time t according to a given function, wherein the integral of the flow F over the time t equals the bolus volume (B).

The term 'bolus administration profile' may be used in the context of a bolus in total but may also be used for at least two sub boli, the at least two sub boli having at least corresponding sub bolus volumes B1, B2 and summing up to the bolus volume B. In this case, the term 'bolus administration sub profile' may be used for clarity reasons for the corresponding bolus administration profiles. It has to be understood, however, that the distribution of a bolus volume B over time t may generally be described by one more complex bolus administration profile instead.

The terms 'immediate bolus' and 'immediate bolus administration profile' refer to a bolus which is administered within a short administration time after receiving a bolus administration command. The administration time is substantially negligible, as compared to the pharmaceutical effect resulting from the drug after administration. The actual administration time for an immediate bolus may range from a fraction of a second to, e.g. 30 sec., dependent factors such as the design of the administration device and the bolus volume of the immediate bolus.

The terms 'extended bolus' and 'extended bolus administration profile' refer to a bolus which is administered over a controlled extended period of time T_extended, the extended period of time T_extended being generally large as compared to the administration time of an immediate bolus. An extended bolus may be administered continuously or may e.g., be administered as a series of extended bolus pulses which make up the extended bolus. The administration of an extended bolus may be started immediately after receiving a bolus administration command or may be started delayed.

The term 'basal profile' refers to the basal administration rate RB over time t. For example, the curve 260 shown in Figure 2a shows a typical basal profile for insulin administration over the time of day in the framework of CSII. The basal profile is generally patient-dependent and is defined by the basal administration rate RB over the time of day. For the example shown in Figure 2a, the basal administration rate RB is varied in four blocks over the day with the basal administration rate RB being identical within each block. The basal administration rate is typically measured in International Units per hour, [IU/h].

The term 'basal administration profile' refers to the actual flow resulting form basal administration over time t. According to a first exemplary basal administration profile, basal administration is performed continuously with the flow caused by basal administration being substantially equal to the basal administration rate RB at any moment of time t. According to further exemplary basal administration profiles, basal administration is performed pulsed. The diagram in Figure 2b illustrates basal administration according to an exemplary pulsed basal administration profile where basal pulses 202, 204 are administered in equal basal pulse intervals dt, with the basal pulse volume P_B of the basal pulses 202, 204 being an equal portion of the basal drug volume which is administered in one hour of day. Figure 2b especially shows a detailed view of detail D in Figure 2a. The basal pulse interval dt may, e.g., be 3min such that each basal pulse 202, 204 administers 1/20th of the basal drug volume which is administered in one hour of day.

It is an objective of the present invention to provide administration devices for the subcutaneous administration of a liquid drug over an extended period of time of time which improve the state of the art with respect to the drawbacks related to insufficient absorption and flow back of administered drug doses and especially of boli as described above without requiring the patient to consider drug absorption.

It is a further objective of the present invention to provide methods for controlling a pump unit of an administration device according to the present invention, the methods improving the state of the art with respect to the drawbacks related to insufficient absorption and flow back of administered drug doses and especially of boli.

Administration devices and methods according to the present invention are of special advantage in the framework of diabetes therapy by CSII, but may also be used in the framework of different therapies such as pain therapy.

The present invention and its preferred and exemplary embodiments are best understood based on an empirical and semi-quantitative absorption model. In the following, this model is given with respect to the administration of a bolus, the bolus having the bolus volume B, but also holds, mutas mutandis, true for subcutaneous administration in a more general context, and especially for combined bolus and basal administration. Steady absorption by the tissue surrounding the cannula tip is only possible for a flow F not exceeding a maximum steady absorption rate R_max, the maximum steady absorption rate R_max typically being in a range of some IU/min in the framework of CSII.

For an actual flow F exceeding this maximum steady absorption rate R_max, the actual absorption rate R_absorption is given by R_max. That is,

$$R\_absorption = \begin{cases} F & \text{if} \quad F \le R\_max \\ R\_max & \text{if} \quad F > R\_max \end{cases} \qquad (1).$$

In the latter case, the administered drug is not fully and immediately absorbed by steady absorption, but is partly stored in a depot and is subsequently delayed from the depot. Instead of the bolus volume B, only a steady absorption bolus volume

$$B\_steady = R\_max \cdot T\_admin \qquad (2)$$

is absorbed by the tissue over the administration time T_admin. Under the assumptions that the term F - R_max is non-negative and that the depot is empty at the beginning of the administration, the resulting depot volume B_depot of the bolus while performing the administration is given as a function of time by

$$B\_depot(t1) = \int_0^{t1} \left[ F(\tau) - R\_max \right] d\tau, \qquad (3a),$$

with t1 running form zero at the beginning of the administration to the administration time T_admin.

If the depot is not empty at the beginning of the administration but contains some initial depot volume B0_depot, resulting, e.g., from a previous administration, the depot volume is given by

$$B\_depot(t1) = \int_0^{t1} \left[ F(\tau) - R\_max \right] d\tau + B0\_depot \qquad (3b).$$

The drug stored in the depot is subsequently delayed absorbed by the tissue from the depot. For practical reasons, the delayed absorption rate is assumed to be constant and substantially equivalent to the maximum steady absorption rate R_max, resulting in the depot volume B_depot to decrease according to a linear function

$$B\_depot(t1) = B\_depot(0) - R\_max \cdot t1 \qquad (4).$$

With t1 starting from zero when ending the administration and B_depot(0) being the depot volume for t1 = 0. The absorption rate, however, may also be different from the maximum steady absorption rate R_max and/or may not be constant.

Establishing a depot results in a temporarily and local tissue pressure increase, the increased tissue pressure leading to mechanical stress which, in extreme cases, may cause local traumatization and/or damage of the tissue.

If the depot volume B_depot exceeds a maximum depot volume B_depot_max, drug flows back along the outer surface of the cannula and out of the tissue. The maximum depot volume B_depot_max is to be dependent on several factors such as the length and design of the administration cannula, and the patient's tissue properties. It is typically in the range of about 5.0 IU to 10.0 IU in the framework of CSII.

An administration device for the subcutaneous administration of a liquid drug over an extended period of time according to the present invention comprises:

a) an administration unit (70), the administration unit (70) comprising a drug reservoir (20) and a pump unit (60),

b) a housing (10), the housing (10) comprising the administration unit (70), the housing (10) being adapted to be carried by a patient over an extend period of time,

c) a controller unit (80), the controller unit (80) comprising

d) a bolus administration profile controller (85), the bolus administration profile controller (85) being adapted to determine a bolus administration profile of a bolus to be administered, the bolus having a bolus volume (B), wherein the administration profile is determined, at least in part, based on the bolus volume (B) such that the depot volume B_depot of a depot is limited.

In the context this invention and especially in the context of a bolus administration profile controller, the terms 'limit' and 'limiting' may refer to limiting the depot volume B_depot of a depot such that the depot volume B_depot does not to exceed a threshold value and/or to lowering a depot volume B_depot of a depot as compared to a depot volume which may be stored in a depot without bolus administration profile controller.

In some preferred embodiments, the controller unit is comprised by the housing along with the administration unit, while in alternatively preferred embodiments, the controller unit is, at least in part, separate from the housing comprising the administration unit and is adapted to communicate with the administration unit via wired interface means such as an electrical connector or wireless interface means such as an infrared or Radio Frequency (RF) data interface.

The controller unit may be realized in general ways and may be designed using different kinds of architectures. It may comprise elements known to the man skilled in the art such as microcontrollers, Application Specific Integrated Circuits (ASICS), program memory and data memory, clock circuits, power circuits, supervision circuits, and the like.

In typical embodiments, the administration device further comprises a power supply, comprising at least one of a one-way battery, a rechargeable battery, a capacitor, or the like.

In preferred embodiments, the pump unit comprises a spindle drive and a rotary motor such as a DC motor, a brushless DC motor, a stepper motor or an electromagnetic intermittent motion device while the drug reservoir comprises a barrel and a piston, the piston being controllable displacable within the barrel by spindle drive in order to perform drug administration. In alternative embodiments, however, the pump unit may be realized according to an alternative pump design known in the art, such as a micro membrane pump or a peristaltic pump and/or the drug reservoir may be a pouch, a bag, or the like. Suitable embodiments for the administration unit are disclosed, among others., in EP0991440, US6248093, US20050238507, or US6736796.

In typical situations, it is generally desirable to keep the administration time T_admin while preventing flowback and limiting the mechanical stress exerted onto the tissue. Thus, determining a bolus administration profile for a given bolus amount B may be formulated as optimization problem. The bolus administration is ideally performed as given by the formulas

$$B\_depot \leq BT\_depot \qquad\qquad (I)$$

$$T\_admin \rightarrow min \qquad\qquad (II),$$

independent of the bolus amount B. That is, determination of a bolus administration profile for a given bolus volume B may be formulated as minimization problem for the administration time T_admin under a boundary condition for the depot volume B_depot.

Most of the embodiments as described in more detail in the following are based on the absorption model given above and/or further simplifications of this absorption model in order to determine parameters and threshold values related to bolus administration. However, other and potentially more sophisticated absorption models may alternatively or additionally be employed. Such an absorption model may, e.g., be based on corresponding equations and/or look-up tables. The absorption model may especially be implemented as code in the controller unit. Alternatively or additionally to an explicit absorption model, the parameters used in the embodiments as described below may be determined empirically, based, e.g., on the experience of a healthcare professional.

For practical purposes, several approximations and assumptions may be used which result in formula (I) and/or formula (II) to be met approximately. For example, limiting the bolus depot amount B_depot may be performed such that the boundary condition (I) is be strictly fulfilled only if the bolus amount B does not exceed a threshold value and/or the administration time T_admin may not be the smallest possible administration time for which the boundary condition is met but may result from a compromise which takes into account further aspects such as implementation complexity.

In preferred embodiments, the bolus administration profile controller 85 is adapted to determine the bolus administration profile such that the depot volume B_depot of a depot does not exceed a depot volume threshold BT_depot. That is, the bolus administration profile is controlled such that the depot volume B_depot is limited to a depot volume threshold BT_depot as maximum depot volume, i.e.,

$$B\_depot(t) \le BT\_depot \qquad\qquad (5).$$

In embodiments where the administration unit is adapted for the substantially continuous administration of a bolus with controllable flow F, based, e.g. on a spindle expressing drug from a cartridge, the spindle being driven by a speed controlled motor, the motor speed may be directly controlled. That is, the administration time T_admin for a given bolus volume B may be controlled to meet the condition given by equation (5), with the bolus depot volume B_depot being computed, e.g., by equation (3a) as given above. The flow F may especially be controlled to be substantially constant and controlled such that depot is filled up to the subcutaneous bolus volume threshold BT_depot while performing the administration.

In embodiments where the administration device is adapted for the administration of bolus pulses with substantially constant bolus pulse intervals ddt between consecutive bolus pulses and a controllable bolus pulse volume P_bolus per pulse, equation (3b) may be used to compute the bolus depot volume B_depot in order to meet the condition given by equation (5). It is especially advantageous to consider the single bolus pulses as separated bolus administrations, with the integral in equation (3b) being equivalent to a bolus pulse volume P_bolus, neglecting the steady absorption while administering the pulses and only considering the delayed absorption following the administration of each bolus pulse.

This kind of pulsed administration is typical for administration devices comprising a pump unit with a motor driven spindle drive, the motor being controlled to start and stop rather than being speed controlled. If, for this kind of embodiment, the bolus pulse interval ddt is sufficiently long for fully absorbing the plus pulse volume P_bolus of a bolus pulse, the initial depot volume B0_depot is zero for each pulse and the bolus pulse volume P_bolus may be controlled to substantially equal the depot volume threshold BT_depot. Otherwise, the current depot volume according, e.g., to equation (4) when administering a bolus pulse is considered as initial depot volume B0_depot for the administration of each bolus pulse. This results in the bolus pulse volume P_bolus being smaller than the depot volume threshold BT_depot.

In embodiments where the administration device is adapted for the administration of bolus pulses, the bolus pulses having a fixed bolus pulse volume P_bolus and a controllable bolus pulse interval ddt between consecutive bolus pulses, the condition of equation (5) may be met in a similar way by controlling the bolus pulse interval ddt. This is the case, for example, if the administration device comprises a solenoid-actuated spindle drive such as the one disclosed in US4562751.

In especially preferred embodiments involving a depot volume threshold BT_depot, the depot volume threshold BT_depot is defined by a maximum depot volume B_depot_max, the maximum depot volume B_depot_max being the maximum depot volume that may be stored in a depot.

In alternatively preferred embodiments, the depot volume threshold BT_depot is smaller than the maximum depot volume B_depot_max in order to reduce the physical stress which is exerted onto the tissue.

In preferred embodiments, the bolus administration profile controller is adapted to determine the bolus administration profile such that the bolus administration profile comprises an immediate bolus administration profile, the immediate bolus administration profile corresponding to an immediate bolus, the immediate bolus having an immediate bolus volume B_immediate.

Depending on the specific embodiment of the administration device, the immediate bolus may be administered according to different bolus administration profiles, e.g. with a substantially continuous flow F, as a single bolus pulse or as a series of bolus pulses.

In especially preferred embodiments involving an immediate bolus, the bolus administration profile controller is adapted to determine the immediate bolus volume B_immediate such that the immediate bolus volume B_immediate does not exceed a bolus volume threshold BT. The bolus volume threshold BT may, e.g., be computed based on a given depot volume threshold BT_depot, using the absorption model as given above.

The bolus volume threshold BT may especially be defined such that the depot volume B_depot of a depot does not exceed a maximum depot volume B_depot_max. Alternatively, the depot volume threshold BT_depot may be defined to be smaller than the maximum depot volume B_depot_max, and to be a given fraction F of the maximum depot volume B_depot max in order to reduce the physical stress which is exerted onto the tissue. The fraction F may, eg., be in the range from 0.5 to 0.8.

In preferred embodiments, the bolus administration profile controller 85 is adapted to determine the bolus administration profile such that the bolus administration profile comprises an extended bolus administration profile, the extended bolus administration profile corresponding to an extended bolus, the extended bolus having an extended bolus volume B_extended.

The extended bolus is administered over an extended period of time T_extended. The extended period of time T_extended, may be a fixed time of, e.g., 15min or 30min or may be adjusted, e.g., based on the extended bolus volume B_extended with the extended period of time T_extended preferably increasing with the extended bolus volume B_extended. For example, a list of at least extended periods of time may be provided and the extended period of time T_extended for administering the extended bolus may be determined to be the smallest value from this list for which the limitation criteria for the depot volume B_depot are met.

In especially preferred embodiments involving an extended bolus, he bolus administration profile controller is adapted to determine the extended bolus as series of extended bolus pulses.

The extended bolus pulses are preferably be administered over the extended period of time T_extended with equal bolus pulse intervals ddt between the administration of the individual extended bolus pulses.

In embodiments where the administration device is adapted for the administration of bolus pulses with controllable bolus pulse volume P_bolus and equal bolus pulse interval ddt, computation of the extended bolus pulse volume P_bolus based on the extended bolus volume B_extended, the extended period of time T_extended and the bolus pulse interval ddt is straight forward. In embodiments where administration device is adapted for the administration of bolus pulses, the bolus pulses having a fixed bolus pulse volume P_bolus and a controllable bolus pulse interval ddt, the bolus pulse interval ddt may be computed based on the extended bolus volume B_extended, the bolus pulse volume P_bolus and the extended period of time T_extended.

In especially preferred embodiments involving the administration of an extended bolus as a series of extended bolus pulses, the bolus administration profile controller is adapted to determine the bolus administration profile such that the bolus pulse volume P_bolus of the extended bolus pulses does not exceed a bolus pulse volume threshold PT_bolus. The bolus pulse volume threshold PT_bolus may, e.g., be computed based on a given depot volume threshold BT_depot, using the absorption model as given above.

The bolus pulse volume threshold PT_bolus may especially be defined such that the depot volume B_depot of a depot does not exceed a maximum depot volume B_depot_max. Alternatively, the depot volume threshold BT_depot may be defined to be smaller than the maximum depot volume B_depot_max, and to be a given fraction F of the maximum depot volume B_depot max in order to reduce the physical stress which is exerted onto the tissue. The fraction F may, e.g., be in the range from 0.5 to 0.8.

If the bolus pulse interval ddt between two consecutive extended bolus pulses is, e.g., 6min, and an administration unit comprising a motor driven spindle drive is used for the administration and the drug is administered via a subcutaneous cannula, the bolus pulse volume threshold PT_bolus may, e.g., be set to 3.0IU, resulting from the assumption that 3.0 IU of subcutaneously administered drug can be delayed absorbed within a period of 3 min.

In preferred embodiments, the bolus administration profile controller is adapted to determine at least a first bolus administration sub profile and a second bolus administration sub profile, and of determining at least a first bolus sub volume and a second bolus sub volume, wherein the at least two bolus administration sub profiles, in combination, form the bolus administration profile and the at least two bolus sub volumes B1, B2 sum up to the bolus volume B. With a first bolus administration sub profile corresponding to an immediate bolus having an immediate bolus volume B_immediate and a second bolus administration sub profile corresponding to an extended bolus having an extended bolus volume B_extended, a suited algorithm may be performed as follows:

```
if B <= BT
    B_immediate = B
    B_extended = 0
else
    B_immediate = BT
    B_extended = B-BT
```

In further similar embodiments, a bolus volume B of a bolus to be administered may be split into an immediate bolus and an extended bolus according to further and more complex algorithms.

In some cases, a patient commands the administration of more than one bolus within a comparatively short period of times. The second bolus administration may be especially commanded and at a time at which the depot is not empty but partly filled due to the first bolus. This is the case, e.g., if the patient administers a bolus before a meal and decides later on that the administered bolus was inappropriately small or if a patient realizes that the volume of a bolus was too small by mistake. In this case, the first bolus is preferably considered for determining the bolus administration profile of the second bolus.

In preferred embodiments, the administration device is adapted to administer at least a first bolus, the first bolus having a first bolus volume (B_I) and a second bolus, the second bolus having a second bolus volume B_II, wherein administration of the first bolus is in progress when the administration of the second bolus is commanded, and in that the bolus administration profile controller (85) is adapted to determine a first administration profile and a further administration profile, wherein the further bolus administration profile is determined based, at least in part, on the first bolus administration profile and/or on the administration state of the first bolus.

That is, the first bolus is taken into account by the bolus administration profile controller for determining the second bolus administration profile of the second bolus such that the depot volume B_depot of a depot does not exceed a depot volume threshold BT_depot. The implementation may, e.g., be performed based on equation (3b) for determining the second bolus administration profile of the second bolus, taking into account the partly filled depot when commanding the administration of the second bolus by the initial depot volume B0_depot. The administration state of the first bolus reflects the portion of the first bolus volume B_I that still has to be delivered when commanding the administration of the second bolus. The further administration profile may only determine the second administration profile of the second bolus of the second bolus, such that the bolus is continued to be administered according the first bolus administration profile or the further administration profile may determine the administration profile of the remaining portion of the first bolus as well as the administration profile of the second bolus.According to further especially preferred type of embodiment, the administration of the second bolus is delayed until the administration of the first bolus is finished.

In preferred embodiments, the administration device is further adapted to perform a basal administration according to a basal profile. This basal profile may especially be a patient-specific and time-of day-dependent basal profile, as the one shown for illustration purposes in Figure 2a. In the framework of diabetes therapy with the drug beeing insulin, the basal administration rate RB may, for example, vary between 0.8 IU/h and 2.0 IU/h over the day. In especially preferred embodiments, basal administration is performed by administering basal pulses. The basal pulses may either be administered with a constant basal pulse interval dt between the basal pulses with the basal pulse volume P_basal being adjusted in accordance with the basal administration rate RB, or may have a constant basal pulse volume P_basal with the basal pulse interval dt being adjusted in accordance with the basal administration rate RB.

In especially preferred embodiments of an administration device being adapted to administer an extended bolus as a series of extended bolus pulses and adapted to perform a basal administration according to a basal profile, the controller unit is adapted to control the administration unit to administer combined pulses, each combined pulse comprising an extended bolus pulse and a basal pulse.

In one type of embodiment, the combined pulses are administered as consecutive pulses with no further basal pulses being administered between the combined pulses. In another type of embodiment, the combined pulses are administered as non-consecutive pulses with the administration of each combined pulse being followed by the administration of at least one basal pulse.

In especially preferred embodiments of administration devices being adapted to administer combined pulses, the bolus administration profile controller is adapted to determine the bolus administration profile such that the combined pulse volume P_combined of the combined pulses does not exceed a pulse volume threshold PT.

The determination of the bolus administration profile such that the combined pulse volume P_combined does not exceed a given pulse volume threshold PT may especially be employed in cases where the basal pulse volume P_basal is not negligible as compared to the bolus pulse volume P_bolus. Because the basal pulse amout P_basal is determined by the basal profile and the basal administration profile, the bolus pulse volume P_bolus is preferably adapted to meet the restriction for P_combined.

The pulse volume threshold P_combined may, e.g., be computed based on a given depot volume threshold BT_depot, using the absorption model as given above.

The pulse volume threshold P_combined may especially be defined such that the depot volume B_depot of a depot does not exceed a maximum depot volume B_depot_max. Alternatively, the depot volume threshold BT_depot may be defined to be smaller than the maximum depot volume B_depot_max, and to be a given fraction F of the maximum depot volume B_depot max in order to reduce the physical stress which is exerted onto the tissue. The fraction F may, e.g., be in the range from 0.5 to 0.8.

In preferred embodiments, the administration device is generally adapted for administering drug pulses, and the bolus administration profile controller is adapted to determine the pulse volume and/or of the pulse interval such that the depot volume B_depot of a depot does not exceed a depot volume threshold BT_depot.

The depot volume threshold P_combined may especially be defined such that the depot volume B_depot of a depot does not exceed a maximum depot volume B_depot_max. Alternatively, the depot volume threshold BT_depot may be defined to be smaller than the maximum depot volume B_depot_max, and to be a given fraction F of the maximum depot volume B_depot max in order to reduce the physical stress which is exerted onto the tissue. The fraction F may, e.g., be in the range from 0.5 to 0.8.

In preferred embodiments, the administration device comprises a user interface, the user interface being coupled to the controller unit, the user interface being adapted to receive a bolus administration command. The preferably present user interface may comprise entry elements such as pushbutton, coordinate switches, a numeric keypad, or the like and/or output elements such as a display. In especially preferred embodiments comprising a display, the display is a Liquid Chrystal Display (LCD) or an Organic Light Emitting Diode (OLED) display. In especially preferred embodiments, the user interface further comprises non-visual indicator elements, such as an acoustic indicator and/or a tactile indicator. In some preferred embodiments comprising a user interface, the user interface is, at least in part, comprised by the housing. In addition or alternative to a user interface comprised by the housing, a remote user interface is provided in preferred embodiments, the remote user interface being coupled to the controller unit via a wireless data interface. A remote user interface may be a fully functional user interface including entry and output elements, or may offer a reduced set of operational functions. A remote user interface offering a reduced set of operational functions may, e.g., be comprised by a key fob like device and may only offer the entry of boli to be administered.

Dependent on the type of user interface, bolus administration commands may be received by the user interface in different ways. In especially preferred embodiments, a bolus volume B may be entered in a bolus entry mode oft the administration device using up and down buttons for increasing and decreasing the bolus volume B, followed by a succeeding confirmation. Alternatively or additionally, a bolus may be entered by multiple keystrokes of a defined key, wherein each keystroke increases the bolus volume B by a given increment in especially preferred embodiments.

In especially preferred embodiments comprising a user interface adapted to receive a bolus administration command, the user interface is adapted to indicate the reception of a bolus command with the administration device being, concealed from view. In especially preferred embodiments, the user interface is further adapted to indicate a received bolus volume B with the administration device being, at least in part, concealed from view. In this kind of embodiment, both indicating the reception of a bolus administration command and indicating the received bolus volume B may be performed via a preferably present acoustic indicator and/or tactile indicator, the acoustic indicator and/or the tactile indicator being control to emit a series of indication pulses, with each indication pulse being representative for a given bolus increment. In further embodiments, the bolus volume B is displayed on a display being comprised by a remote user interface.

In preferred embodiments, the administration device controller is adapted to be selectively deactivated. In especially preferred embodiments, the preferably present user interface allows to manually select a bolus administration profile and the bolus administration profile controller is deactivated if a bolus administration profile for a bolus to be administered is manually selected, wherein the selected bolus administration profile preferably is a member of a set of defined bolus administration profiles stored by the controller.

In especially preferred embodiments comprising a bolus administration profile controller which is adapted to be selectively deactivated, the user interface offers at least two different ways of receiving a bolus administration command and the bolus administration profile controller is adapted to be selectively deactivated in dependence on the way the bolus administration command is received by the user interface. For example, the bolus administration profile controller may generally be deactivated if a bolus administration command is received by a first user interface which is integral with the administration device and offers direct input of a desired bolus administration profile while the bolus administration profile controller may be activated if a bolus administration command is received from a second user interface, the second user interface being, e.g., a key fob like remote user interface which offers only bolus volume input capabilities. In a similar way, the bolus administration profile controller may be activated if a bolus administration command is received from a portion of the user interface which is integral with the administration device housing and is especially adapted to be operated with the administration device being concealed from view and according only offering access to a limited set of functions.

In especially preferred embodiments allowing selectively deactivating the bolus administration profile controller, the bolus administration profile controller may be generally deactivated by a healthcare professional and/or the patient by a user interface and/or a data interface preferably comprised by the administration device.

A method for controlling a pump unit of an administration device, the administration device being adapted for the subcutaneous administration of a liquid drug over an extended period of time according to the present invention comprises the steps of:

a) providing a bolus volume (B) of a bolus to be administered,

b) determining by a bolus administration profile controller (85) a bolus administration profile of the bolus to be administered, at least in part, based on the bolus volume (B), such that the depot volume B_depot of a depot is limited, and

c) controlling the pump unit (60) to administer the bolus according to the bolus administration profile determined by the bolus administration profile controller (85).

The method may be carried out using administration devices according to the present invention as described above.

In preferred embodiments, the method further comprises the step of providing a drug reservoir, the drug reservoir being filled with insulin. This kind of embodiment is especially suited for the diabetes therapy via CSII.

In especially preferred embodiments, the method comprises the step of determining the bolus administration profile partly based on the pharmacokinetics of the insulin and/or on food absorption characteristics.

For different types of insulin having different pharmacokinetics, different bolus administration profiles may be appropriate and may accordingly be taken into account in addition to the bolus volume B for determining the bolus administration profile. For example, for a slow-acting insulin, a shorter period of time may be appropriate for the bolus administration as compared to a very short acting insulin analogue. Furthermore, for different kinds of food different bolus administration profiles may be appropriate and the food type may accordingly be taken into account in addition to the bolus volume B for determining the bolus administration profile. For example, for a meal comprising considerable volumes of both fat and carbohydrates, such as pizza, a longer period of time may be appropriate for the bolus administration as compared to a meal comprising mainly carbohydrates, such as most fruits or Spaghetti. For entering food absorption characteristics, a food database may be provided, a semi-quantitative selection option, such as 'slow absorption', 'medium absorption' or 'fast absorption' may be provided or the entry of at least one numerical value, the numerical value characterizing the absorption characteristics, may be foreseen at the administration device employed for carrying out the method according to the present invention.

In further especially preferred embodiments, the method comprises the step of determining the bolus volume B based, at least in part, on a blood glucose value of a diabetic person. An insulin bolus may be administered in order to lower an undesirably high blood glucose value. Blood glucose values may be determined e, g, at specific points in time using commercially available blood glucose meters such as ACCU-CHECK® Complact plus or ACCU-CHECK® Aviva, both available from Roche Diagnostics and/or on a continuous or quasi-continuous basis by a continuous blood glucose monitor as known in the art. Based on the physiological characteristics and potential further input, such as carbohydrate intake, a bolus volume may be determined according to different methods known in the art. A variety of methods are known in the art for computing the bolus volume B of an insulin bolus based on blood glucose measurements, such as the disclosure in WO2007056592.

Further preferred embodiments of the method according to the present invention are obvious form the description of preferred embodiments of administration devices according to the present invention as described above.

In the following, exemplary embodiments of administration devices according to the present invention and exemplary embodiments of methods for controlling the administration of a liquid bolus according to the present invention are described in greater detail with reference to the figures.

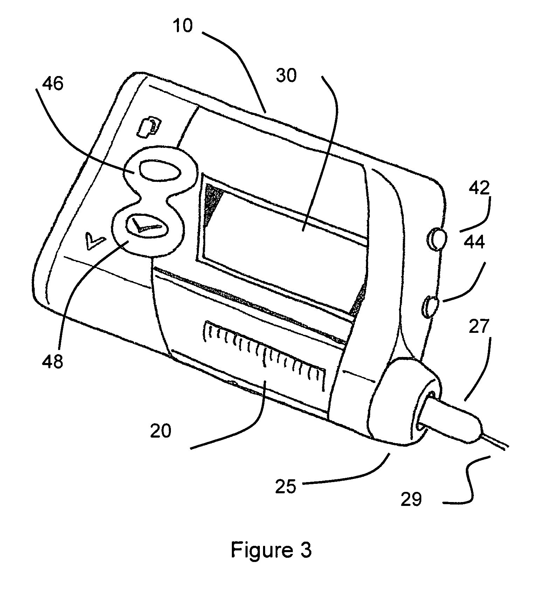

Figure 3 shows an administration device according to an exemplary embodiment of the invention.

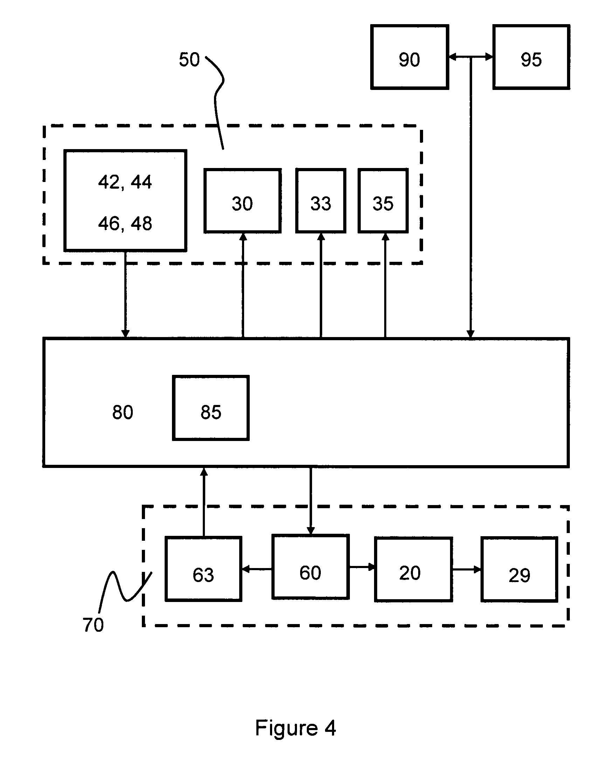

Figure 4 shows the internal functional structure of the administration device shown in Figure 3.

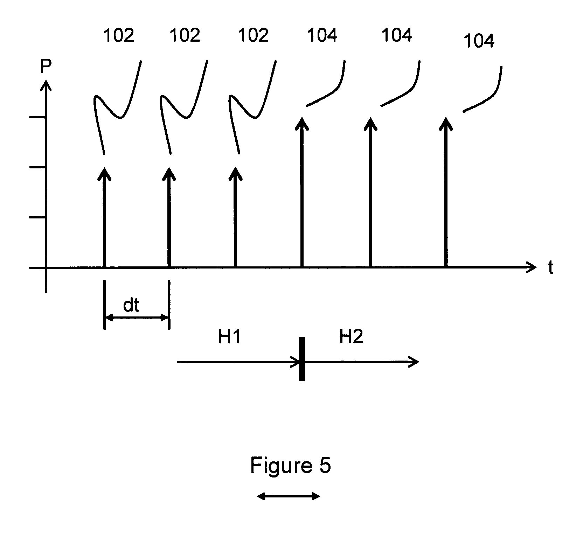

Figure 5 shows an exemplary basal administration profile for basal administration.

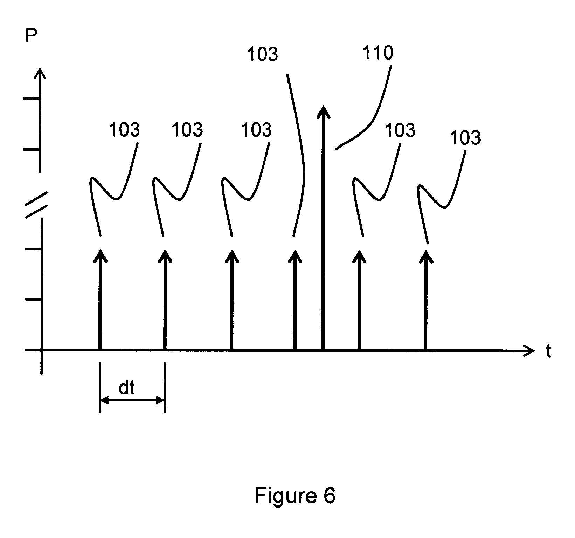

Figure 6 shows an exemplary administration profile comprising basal administration as well as bolus administration, the bolus volume being below a bolus volume threshold according to a first exemplary embodiment of an administration device and an administration method according to the invention.

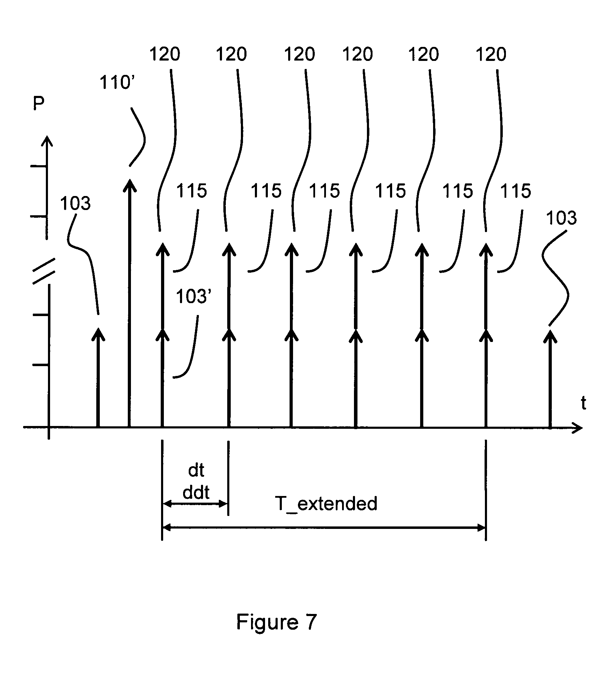

Figure 7 shows an exemplary administration profile comprising basal administration as well as bolus administration, the bolus volume being above a bolus volume threshold according to the first exemplary embodiment of an administration device and an administration method according to the invention.

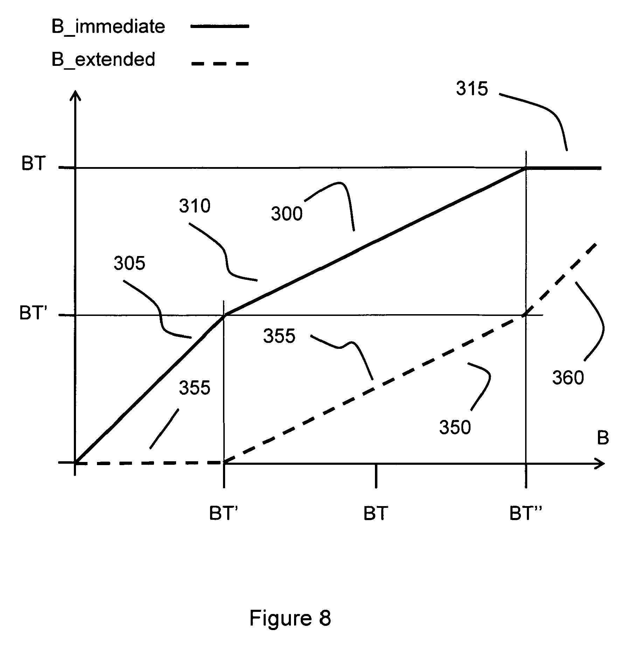

Figure 8 shows the splitting of a bolus into an immediate bolus and an extended according to a second exemplary embodiment of an administration device and an administration method according to the invention.

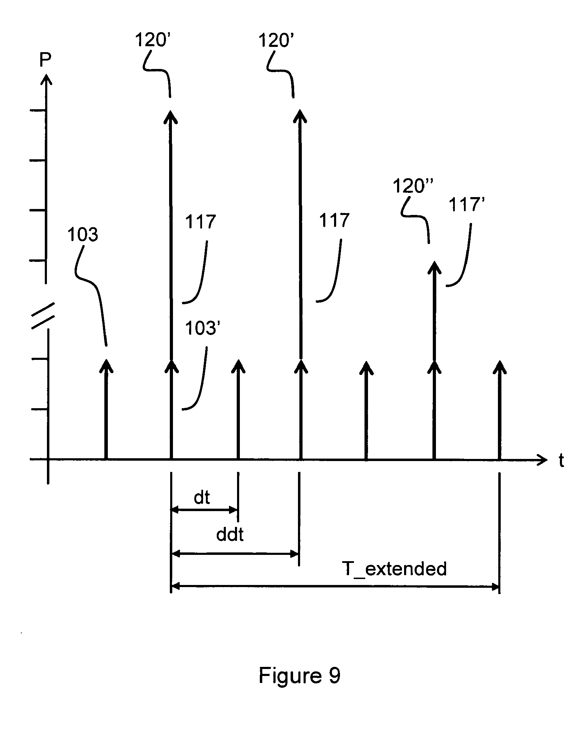

Figure 9 shows an exemplary administration profile comprising basal administration as well as bolus administration, the bolus volume being above a bolus volume threshold according to a third exemplary embodiment of an administration device and an administration method according to the invention.

Figure 3 shows an administration device according to exemplary embodiments of the invention, Figure 4 reflects the internal structure of the administration device. This exemplary embodiment is especially suited and designed for the insulin administration in the framework of diabetes therapy using CSII and the following description of exemplary embodiments refers to diabetes therapy. However, the device may be used for other therapies, such as pain therapy or cancer therapy with minor modifications, if any. In the following, reference is first made to Figure 3 and to Figure 4. Whereas the bolus administration profile controller is different for each of the exemplary embodiments described below, the administration device may be in accordance with the Figure 3 and Figure 4 and the general description as given in the following for all exemplary embodiments.

The administration device comprises a housing 10, the housing 10 enclosing the other device components. The housing 10 is adapted to be virtually permanently worn by a patient both night and day and to be carried concealed from view, i.e., without attracting attention It may be carried in a belt holster, in a trouser pocket, like a necklace, or the like.

The administration device comprises a drug reservoir 20, an infusion line 29 and an infusion line adapter 25, the infusion line adapter coupling the infusion line 29 to the drug reservoir 20. The housing 10, the drug reservoir 20 and the infusion line adapter 25 are designed to form a sealed and watertight unit in the assembled state shown in Figure 3. The infusion line 29 is connected to an infusion cannula (not visible in the figures). The infusion cannula is placed in the subcutaneous tissue and is replaced by the patient every few days. The drug reservoir 20 may, for example, comprise 3.15ml of insulin, with each ml of insulin corresponding to 100 International Units (IU) of insulin.

The exemplary administration devices comprises a user interface 50, the user interface 50 comprising four pushbuttons 42, 44, 46, 48, a display 30, an acoustic indicator 33 in form of a buzzer and a tactile indicator 35 in form of a pager vibrator. The display 30 is a graphical Liquid Chrystal Display (LCD) and is used to provide multiple kinds of information well known for this kind of device, such as operation mode information, time and date, bolus information, current basal infusion rate information, menu items for programming the administration device, alerts and error messages, and the like. The acoustic indicator 33 and the tactile indicator 35 are provided for warning and alarming purposes as well as well as for providing non-visual user feedback on user operations if the display 30 is not comfortable and discretely accessible because the administration device is, e.g., carried under the cloths.

For everyday operation purposes, the four pushbuttons 42, 44, 46, 48 are provided. The Menu pushbutton 46 and the Select pushbutton 48 are arranged on the front face of the housing 10. The Menu pushbutton 46 is used to access different data entry and programming menus and the Select pushbutton is uses to accept and confirm different kinds of entries. Two side face pushbuttons 42, 44 are arranged at a side face of the housing 10 next to the infusion line adapter 25. The side face pushbuttons 42, 46 are especially designed to be easily and securely operated even if they are not visible and may be operated through a layer of cloths, or the like. Therefore, the side face pushbuttons 42, 44 project with respect to the housing 10 to be easily sensed and provide tactile force feedback when being operated. The side face pushbuttons 42, 44 are used for selecting items from a menu, for increasing/decreasing numeric values during entry and for commanding the administration of boli as will be described below in more detail.

For performing the drug administration, a pump unit 60 and a supervision unit 63 are provided. In this exemplary embodiment, the pump unit 60 comprising a motor-driven spindle drive and the supervision unit 63 comprises a force sensor and a rotary encoder. In combination, the drug reservoir 20, the pump unit 60 and the supervision unit 63 form the administration unit 70. Several suitable designs for the administration unit 60 and the pump unit 70 are known in art and may be designed, for example according to the disclosure of EP1124600B1 or EP0991440B1.

The operation of the administration device is controlled by the controller unit 80. The Controller unit 80 is realized as an electronics circuit and comprises all the elements typically comprised by the control circuit of this kind of administration devices, such as one or multiple micro controllers, static and dynamic memory, a clock circuit, a power circuit for controlling the pump unit 60, safety circuits, and the like. The controller unit is connected to a power supply such as a rechargeable or non-rechargeable battery.

For a pump unit comprising a motor-driven spindle drive, administration is controlled by controlling the motor shaft rotation. While only a fraction of a rotation of the motor shaft may be performed for the administration of small pulses, the administration of a larger drug volume results in multiple rotations of the rotary motor shaft. During the administration of larger drug volumes with virtually continuous operation of the rotary motor of the pump unit 60, the administration rate is typically performed with a substantially steady administration rate and a follow F of, e.g., 0.25IU/sec. No steady state is taken for the administration of small drug volumes and the flow F at the cannula is pulse like in this latter case. In the following, however, each drug administration is considered as Dirac pulse of negligible duration and controlled drug volume for modeling and representation purposes in accordance with the definition given above..

The administration device further comprises two bidirectional data interfaces, namely an Infra Red IR data interface 90 and an Radio Frequency RF data interface 95, according, e.g, to the BLUETOOTH standard. The IR data interface 90 is especially suited for data exchange with virtually every external standard device, such as a PC or a Personal Digital Assistant PDA and may be used, e.g., for downloading history data to an external device or for uploading configuration data to the administration device. The RF data interface 95 may be used, e.g., for transferring remotely generated administration commands to the administration device and for further remote control purposes.

The administration device may be operated in either of an administration mode and a suspend mode, wherein no drug is administered if the administration device is operated in the suspend mode. The suspend mode is chosen, e.g., for replacing the drug reservoir 20, when temporarily removing the administration device, e.g., during sportive activities for changing configuration parameters of the administration device. The administration device may further change from the administration mode into the suspend mode autonomously, e.g. in the case of an administration device failure.

Most of the time, however, the administration device is operated in the administration mode. In this mode, the administration device is adapted for drug administration according to two administration regimes, the two administration regimes being overlaid and being carried out simultaneously.

The first of the two administration regimes is a basal administration regime. According to the basal administration regime, the controller unit 80 controls the pump unit 60 to administer drug according to a basal profile. In the framework of diabetes therapy, the basal administration covers the patient's basal, i.e., meal independent, insulin demand for maintaining the patient's metabolism. In this framework, the basal profile typically shows a 24h-cycle with the administration rate being constant within each hour of a day and being generally different for different hours of the day. The basal profile may be configured either using the user interface 50 of the administration device or via either of the data interfaces 90, 95.

The basal administration profile is illustrated in Figure 5, resulting in a quasi-continuous administration Comparatively small basal pulses 102, 104 are administered with a fixed basal pulse interval dt. The administration pulse interval may, e.g., be 3 min. This results in the basal pulse volume P_basal being 1/20 of the total hourly basal drug volume. The basal pulses 102 are administered at fixed points in time. With the basal pulse interval dt being 3 min, a basal pulse 102 is administered every full hour, 3 min after the full hour, 6 min after the full hour and so on. In similar embodiments, the basal pulses may be administered at different points of time and/or with a different basal pulse interval dt of, e.g., 6 min or 15 min.

In the example shown in Figure 5, the first three basal pulses 102 belong to a first hour of day H1, while the second three basal pulses 104 belong to a succeeding second hour of day H2, with the administration rate in the second hour of day H2 being 1.5 times the administration rate in the first hour of day H1. For example the administration rate in the first hour of day H1 may be 0.8 IU/hr and the administration rate in the second hour of day H2 may be 1.2 IU/hr.

The second administration regime is a bolus administration regime. If the exemplary administration device is used in the framework of diabetes therapy, the administration of insulin boli is required in order to cover the intake of carbohydrates and in order to lower undesirably high blood glucose values. While spreading bolus administration over an extended period of time may bee desirable for pharmacokinetic reasons in some cases, it is normally desirably to perform bolus administration within a short period of time. Bolus administration according to the second administration regime may be performed at any point in time while continuing the basal administration according to the first administration regime.

For commanding the administration of a bolus, different options are provided. According to a first method for commanding the administration of a bolus, a standard bolus administration menu may be activated using the Menu pushbutton 46 and the Select pushbutton 48. In this standard bolus administration menu, a bolus volume B to be administered may be increased and decreased via the side face pushbuttons 42, 44. During entering the bolus volume B, it is shown on the display 30. After finishing the entry, it is confirmed by pressing the Select pushbutton 48. This method of commanding the administration of a bolus requires access to all four of the pushbuttons 42, 44, 48, 48 as well as to the display 30.

A second method for commanding the administration of a bolus only requires the operation of the side face pushbuttons 42, 44 and does not require the display 30 to be visible. According to this method, either of the side face pushbuttons 42, 44 is continuously operated for a time of, e.g., 3 sec in order to enter a blind bolus entry mode and the bolus volume B to be administered may be entered by operating the other one of the two side face pushbuttons 42, 44 consecutively a number of times, wherein the bolus volume B to be administered is increased with each keystroke by a given bolus increment of, e.g., 0.5IU. Acoustic and/or tactile feedback are provided to confirm correct entry of the bolus volume B.

Prior to controlling the pump unit 60 to administer the bolus, an appropriate bolus administration profile is determined by the bolus administration profile controller 85. According to the exemplary embodiments described here, drug absorption is considered following the empirical model as given above. However, further physical absorption models may be used, too, resulting in alternative embodiments of the bolus administration profile controller (85).

If the bolus volume B of a bolus to be administered is below a given bolus volume threshold BT, the bolus administration profile controller 85 determines the bolus administration profile to substantially be an immediate bolus pulse 110. Figure 6 shows an administration profile comprising a basal administration profile with basal pulses 103 and an immediate bolus pulse 110. The bolus volume threshold BT is stored in a memory of the controller unit 80 and may be altered via the user interface 80 or either of the data interface 90, 95 by the patient and/or a healthcare professional. Administering the bolus as immediate bolus results in the bolus volume B being temporarily stored in a depot and being delayed administered from the depot.

The bolus volume threshold BT is typically configured to be in the range of some International Units and may, e.g., equal 7.0 IU for a steel infusion cannula which is set into the tissue transversally to the skin and a steady state flow of 0.25 IU/sec. As a rule of thumb, the bolus volume threshold BT in IUs equals the cannula length in mm for a steel canula which is transversally set into the skin. More generally, the bolus value threshold BT is configured such that the bolus depot volume B_depot resulting from the bolus administration does not exceed a given depot volume threshold BT_depot and especially does not exceed a maximum depot volume B_depot_max.

If the bolus volume B to be administered exceeds the bolus volume threshold BT, the bolus administration profile controller 85 determines a bolus administration profile according to Figure 7 with the bolus volume B being split into a first bolus sub volume B1 and a second bolus sub volume B2. The first bolus sub volume B 1 is administered according to a fist bolus administration sub profile while the second bolus sub volume is administered according to a second bolus administration sub profile. The first bolus administration sub profile is an immediate bolus administration profile while the second bolus administration sub profile is an extended bolus administration profile, resulting in the first bolus sub volume B1 being an immediate bolus volume B_immediate and the second bolus sub volume being an extend bolus volume B_extended, wherein

$$B\_extended = B - B\_immediate.$$

The immediate bolus volume B_immediate equals the bolus volume threshold BT and is administered as immediate bolus pulse 110' as described above. The extended bolus volume B_extended is divided into a number of extended bolus pulses 115, each individual extended bolus pulse 115 having an identical bolus pulse volume P_bolus. The extended bolus pulses 115 are administered along with a series of consecutive basal pulses 103', resulting in combined pluses 120 being administered at fixed points in time with each combined pulse 120 comprising an extended bolus pulse 115 and a basal pulse 103', the bolus pulse interval ddt being equal to the basal pulse interval dt. The basal pulses 103' of the combined pulses 120 are determined by the basal administration profile and have the same basal pulse volume P_basal as the basal pulses 103 which are not administered in combination with an extended bolus pulse 115. The extended period of time T_extended may be set by the patient and/or a healthcare professional as configuration parameter and may have a value, of, e.g., 15min or 30min. It generally is a multiple of the bolus pulse interval ddt. According to slightly different embodiments, at least two different extended periods oft time, T_extended_1, and T_extended_2 are provided as configuration parameters and the extended period of time T_extended for administering the extended bolus is selected among T_extended_1 and T_extended_2 in dependence on the extended bolus volume B_extended. More generally, the extended period of time T_extended may be determined such that the bolus depot volume B_depot resulting from the bolus administration does not exceed a maximum depot volume threshold B_depot_max and especially does not exceed a maximum depot volume B_depot max.

In many cases however, the combined pulse volume P_combined of the combined pulses 120 is sufficiently small and the bolus pulse interval ddt is sufficiently large to ensure absorption of a combined pulse 120 in the interval ddt if the extended period of time is set to a fixed value of, e.g., 30min.

The bolus pulse volume P_bolus of each extended bolus pulse 115 may be computed according to the equation

$$P\_bolus = B\_extended\ /\ (T\_extended/ddt + 1)$$

for a uniform distribution of the extended bolus volume B_extended onto all combined pulses 120. The denominator of this equation equals the number of combined pulses. The first of the extended bolus pulses 115 is administered along with the first basal pulse 103' after commanding the administration of the bolus.

In slightly modified embodiments, the immediate bolus is administered after administering the extended bolus or the immediate bolus is administered during administering the extended bolus.

In some cases it may be desired to manually determine the bolus administration profile independent of the bolus administration profile controller 85, e.g., for taking into account the glycemic response of special food types of complex glycemic characteristics and especially slow carbohydrate absorption, such as menue dinner where the bolus administration over an extended period of time T_extended may be appropriate even for a bolus volume B below the bolus volume threshold BT. For this purpose, dedicated bolus administration profiles may be manually selected via the user interface 50. In somewhat modified embodiments, the bolus administration profile controller 85 determines the bolus administration profile taking into account both bolus volume B and glycemic food characteristics.

The controller unit 80 may further be configured to activate the bolus administration profile controller 85 only if the side face pushbuttons 42, 44 and/or either of the data interfaces 90, 95 are used for commanding the bolus administration and entering the bolus volume but to deactivate the bolus administration profile controller 85 if the menu pushbutton 46 and the select pushbutton 48 are used. This is advantageous because commanding a bolus administration via the side face push buttons 42, 44 shall often be performed quickly and without access to all elements of the user interface. In this kind of embodiment, a bolus administration profile which is appropriate in most cases is automatically selected, while the patient may also decide to have full manual control over the bolus administration profile if required.

The controller unit 80 may further be configured to generally deactivate the bolus administration profile controller 85.

The diagram shown in Figure 8 shows the splitting of a bolus volume B into an immediate bolus volume B_immediate and an extended bolus volume B_extended according to a second exemplary embodiment. According to this embodiment, splitting the bolus volume B into an immediate bolus volume B_immediate and an extended bolus volume B_extended is not performed only if the bolus volume B exceeds the bolus volume threshold BT but is performed for a bolus volume B exceeding a bolus splitting threshold BT', the bolus splitting threshold BT' being smaller than the bolus volume threshold BT. As seen from the solid line 300 representing the immediate bolus volume B_immediate, the immediate bolus volume B_immediate equals the bolus volume B for the bolus volume B being smaller than the bolus splitting threshold BT', while the extended bolus volume B_extended, as being represented by the dashed line 350, is zero in this case. For a bolus volume B exceeding the bolus splitting threshold BT', the portion B' of the bolus volume which exceeds the bolus splitting threshold BT' is split to contribut in equal parts to the immediate bolus volume B_immediate and an extended bolus volume B_extended, as shown by the segments 310, 360 of equal slopes. This is

$$B' = B - BT'$$

$$B\_immediate = BT' + B'/2$$

$$B\_extended = B'/2$$

In the example shown in Figure 8, the bolus splitting threshold BT' is half the bolus volume threshold BT. However, other values may be used, too.

A limiting bolus volume threshold BT" is defined as the bolus volume B for which the immediate bolus volume B_immediate equals the bolus volume threshold BT. As may be seen from the segments 315 and 365, respectively, the immediate bolus volume B_immediate does not further increase for the bolus volume B increasing beyond the limiting bolus volume threshold BT", while the bolus volume exceeding the limiting bolus volume threshold BT" only adds to the extended bolus volume B_extended. For this exemplary embodiment, the limiting bolus volume threshold BT" equals 3/2*BT. For the bolus volume B exceeding the limiting bolus volume threshold BT" the administration is identical to the first exemplary embodiment of the method for controlling bolus administration.

This second exemplary embodiment is advantageous in so far as the transition between the administration of an immediate bolus only and the administration of both an immediate bolus and an extended bolus is more smoothly as compared to the first exemplary embodiment. It is obvious for the man skilled in the art that other threshold values than those shown in Figure 8 may be used or that the splitting may be performed according to different algorithms in a similar way. For calculating the immediate bolus volume B_immediate and the extended bolus volume B_extended as a function of the bolus volume B, the bolus administration profile controller 85 may directly compute the immediate bolus volume B_immediate and the extended bolus volume B_extended according to an equation or may employ a look-up table.

Figure 9 illustrates the administration of a bolus exceeding the bolus volume threshold BT according to a third exemplary embodiment. According to this third exemplary embodiment, the bolus administration profile controller 85 determines a bolus administration profile such that the bolus is split into a number of extended bolus pulses 117, 117' which are administered along with a series of non-consecutive basal pulses 103'. This bolus administration profile results in the administration of a multiple sequence of a combined pulse 120', 120", each combined pulse 120' being followed by at least on basal pulse 103. In the example of Figure 6, each combined pulse 120' is followed by one basal pulse 103, resulting in the bolus pulse interval ddt being double the basal pulse interval dt. The extended bolus pulse volume P_bolus of the extended bolus pulses 117 equals a bolus pulse volume threshold PT_bolus and the number n_bolus of extended bolus pulses 117, 117' may be computed according to the equation

$$n\_bolus = ceil(B / PT\_bolus),$$

with 'ceil' indicating integral rounding up. Because the bolus volume B is generally not an integral multiple of the bolus pulse volume threshold PT_bolus, the last extended bolus pulse 117' is typically smaller than the preceding extended bolus pulses 117 resulting in the volume last combined pulse 120" being accordingly smaller than of the preceding combined pulses 120'. In a slightly modified embodiment, the bolus administration profile controller 85 determines the bolus administration profile such that the extended bolus pulse volume P_bolus is equal for all extended bolus pulses 117, 117'.

In this exemplary embodiment, the bolus pulse volume threshold PT_bolus is determined such that it is absorbed in a time period ddt = 2 * dt by delayed absorption whereas the basal pulse volume P_basal of the basal pulses 103 is small enough not to be considered with respect to absorption.

**Claims**

1. Administration device for the subcutaneous administration of a liquid drug over an extended period of time, comprising:

   a) an administration unit (70), the administration unit (70) comprising a drug reservoir (20) and a pump unit (60),
   b) a housing (10), the housing (10) comprising the administration unit (70), the housing (10) being adapted to be carried by a patient over an extend period of time,
   c) a controller unit (80),
   **characterized in that** the controller unit (80) further comprises
   d) a bolus administration profile controller (85), the bolus administration profile controller (85) being adapted to determine a bolus administration profile of a bolus to be administered, the bolus having a bolus volume (B), wherein the administration profile is determined, at least in part, based on the bolus volume (B) such that the depot volume B_depot of a depot is limited.

2. Administration device according to Claim 1, **characterized in that** the administration device further comprises a user interface (50), the user interface (50) being coupled to the controller unit (80), the user interface (50) being adapted to receive a bolus administration command.

3. Administration device according to Claim 2, **characterized in that** the user interface (50) is adapted to indicate the reception of a bolus administration command with the administration device being concealed from view.

4. Administration device according to either of Claim 1 to Claim 3, **characterized in that** the bolus administration profile controller (85) is adapted to be selectively deactivated.

5. Administration device according to Claim 4 and according to either of Claim 2 or claim 3, **characterized in that** the user interface offers at least two different ways of receiving a bolus administration command and that the bolus administration profile controller (85) is adapted to be selectively deactivated in dependence on the way the bolus administration command is received by the user interface (50).

6. Administration device according to either of the previous claims, **characterized in that** the bolus administration profile controller (85) is adapted to determine the bolus administration profile such that the depot volume (B_depot) of a depot does not exceed a depot volume threshold (BT_depot).

7. Administration device according to Claim 6, **characterized in that** the depot volume threshold (BT_depot) is defined by a maximum depot volume B_depot_max, the maximum depot volume B_depot_max being the maximum depot volume that may be stored in a depot.

8. Administration device according to either of the previous claims, **characterized in that** the bolus administration profile controller (85) is adapted to determine the bolus administration profile such that the bolus administration profile comprises an immediate bolus administration profile, the immediate bolus administration profile corresponding to an immediate bolus, the immediate bolus having an immediate bolus volume (B_immediate).

9. Administration device according to Claim 8, **characterized in that** the bolus administration profile controller (85) is adapted to determine the immediate bolus volume (B_immediate) such that the immediate bolus volume (B_immediate) does not exceed a bolus volume threshold (BT).

10. Administration device according to either of the previous claims, **characterized in that** the bolus administration profile controller (85) is adapted to determine the bolus administration profile such that the bolus administration profile comprises an extended bolus administration profile, the extended bolus administration profile corresponding to an extended bolus, the extended bolus having an extended bolus volume (B_extended).

11. Administration device according to Claim 10, **characterized that** the bolus administration profile controller (85) is adapted to determine the extended bolus as series of extended bolus pulses (115, 117, 117).

12. Administration device according to Claim 11, **characterized in that** the bolus administration profile controller (85) is adapted to determine the bolus administration profile such that the bolus pulse volume (P_bolus) of the extended bolus pulses (115, 117, 117') does not exceed a bolus pulse volume threshold (PT_bolus).

13. Administration device according to either of the previous claims, **characterized in that** the administration device is further adapted to perform a basal administration according to a basal profile.

14. Administration device according to either of Claim 11 or Claim 12 and according to Claim 13, **characterized in that** controller unit (80) is adapted to control the administration unit (80) to administer combined pulses (120, 120', 120"), each combined pulse (120, 120', 120") comprising an extended bolus pulse (115, 117, 117') and a basal pulse (103', 103').

15. Administration device according to Claim 14, **characterized in that** the bolus administration profile controller (85) is adapted to determine the bolus administration profile such that the combined pulse volume (P_combined) of the combined pulses (120, 120', 120") does not exceed a pulse volume threshold (PT).

16. Administration device according to either of the previous claims, **characterized in that** the bolus administration profile controller (85) is adapted to determine at least a first bolus administration sub profile and a second bolus administration sub profile, and of determining at least a first bolus sub volume (B1) and a second bolus sub volume (B2), wherein the at least two bolus administration sub profiles, in combination, form the bolus administration profile and the at least two bolus sub volumes (B1, B2) sum up to the bolus volume B.

17. Administration device according to either of the previous claims, **characterized in that** the administration device is adapted for administering drug pulses, and **in that** the bolus administration profile controller is adapted to determine the pulse volume (PT_bolus, P_extended, P_co,bined) and/or of the pulse interval (dt, ddt) such that the depot volume (B_depot) of a depot does not exceed a depot volume threshold (BT_depot).

18. Administration device according to either of the previous claims, **characterized in that** the administration device is adapted to administer at least a first bolus, the first bolus having a first bolus volume (B_I) and a second bolus, the second bolus having a second bolus volume B_II, wherein administration of the first bolus is in progress when the administration of the second bolus is commanded, and **in that** the bolus administration profile controller (85) is adapted to determine a first administration profile and a further administration profile, wherein the further bolus administration profile is determined based, at least in part, on the first bolus administration profile and/or on the administration state of the first bolus.

19. Method for controlling a pump unit (60) of an administration device, the administration device being adapted for the subcutaneous administration of a liquid drug over an extended period of time, the method comprising the steps of:

> a) providing a bolus volume (B) of a bolus to be administered,
> b) determining by a bolus administration profile controller (85) a bolus administration profile of the bolus to be administered, at least in part, based on the bolus volume (B), such that the depot volume B_depot of a depot is limited, and
> c) controlling the pump unit (60) to administer the bolus according to the bolus administration profile determined by the bolus administration profile controller (85).

20. Method according to Claim 19, **characterized in that** the method further comprises the step of providing a drug reservoir (20), the drug reservoir (20) being filled with insulin.

21. Method according to Claim 20, **characterized in that** the method comprises the step of determining the bolus administration profile partly based on the pharmacokinetics of the insulin and/or on food absorption characteristics.

22. Method according to either of Claim 20 or Claim 21, **characterized in that** the method comprises the step of determining the bolus volume (B) based, at least in part, on a blood glucose value of a diabetic person.

Prel

1

210

t

Figure 1a

Prel

1

215    215    215

1/3

ddt

t

Figure 1b

215    215    215    215    215

Prel

1/5

ddt

t

Figure 1c

F

250

F0

T_admin

Figure 1d

RB

D

260

6    12    18    24    t

Figure 2a

P    202    202    202    204    204    204

dt

t

Figure 2b

Figure 3

Figure 4

Figure 5

Figure 6

Figure 7

Figure 8

Figure 9

**European Patent Office**

## PARTIAL EUROPEAN SEARCH REPORT

which under Rule 63 of the European Patent Convention
shall be considered, for the purposes of subsequent
proceedings, as the European search report

Application Number

EP 08 00 3164

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/160683 A1 (BLOMQUIST MICHAEL L [US]) 28 August 2003 (2003-08-28) <br> * abstract * <br> * figures 1,2,14,17,25,27-30 * <br> * paragraphs [0028] - [0037], [0045] - [0048], [0113] - [0115], [0123], [0132], [0148] - [0168], [0175], [0179], [0182] * <br> * paragraphs [0183], [0198] - [0202], [0211] - [0218], [0226] - [0229], [0248] * | 1-5,8-18 | INV. <br> G06F19/00 <br><br> ADD. <br> A61M5/142 |
| X | ----- <br> WO 2006/124716 A (UNIV BOSTON [US]; DAMIANO EDWARD [US]; EL-KHATIB FIRAS [US]) 23 November 2006 (2006-11-23) <br> * abstract * <br> * figures 1-4 * <br> * page 1, line 25 - page 4, line 12 * <br> * page 8, line 6 - page 10, line 10 * <br> * claims 1,4,7-9,11,13,15,18,27 * <br> ----- <br> -/-- | 1,2,6-9, 13,17 | |

TECHNICAL FIELDS SEARCHED (IPC)

A61M
G06F

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 4 July 2008 | Petersch, Bernhard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent** 
**Office**

**PARTIAL EUROPEAN SEARCH REPORT**

Application Number

EP 08 00 3164

| | DOCUMENTS CONSIDERED TO BE RELEVANT | | CLASSIFICATION OF THE APPLICATION (IPC) |
|---|---|---|---|
| Category | Citation of document with indication, where appropriate, of relevant passages | Relevant to claim | |
| X | US 2003/114836 A1 (ESTES MARK C [US] ET AL) 19 June 2003 (2003-06-19)<br><br>* abstract *<br>* figures 1-5 *<br>* paragraphs [0007], [0009], [0011], [0013], [0026] - [0033], [0046] - [0051], [0065] - [0070], [0074], [0082] - [0085] *<br>----- | 1,2, 8-10,13, 16 | |
| X | WO 2006/089965 A (NOVO NORDISK AS [DK]; KRISTENSEN JOERGEN SMEDEGAARD [DK]) 31 August 2006 (2006-08-31)<br>* abstract *<br>* figures 10-20 *<br>* page 1 - page 5 *<br>* page 7 - page 9 *<br>* page 13 - page 14 *<br>* page 16 - page 17 *<br>----- | 1,2,8, 10,13,16 | |
| A | EP 1 338 295 A (LIFESCAN INC [US]) 27 August 2003 (2003-08-27)<br>* abstract *<br>* figures 3A,3B,3C *<br>* paragraphs [0011], [0012], [0029] - [0031], [0040], [0065] - [0071] *<br>----- | 1-18 | TECHNICAL FIELDS SEARCHED (IPC) |

EPO FORM 1503 03.82 (P04C10)

Claim(s) not searched:
    19-22

Reason for the limitation of the search (non-patentable invention(s)):

Article 53 (c) EPC - Method for treatment of the human or animal body by therapy: claim 19 (and thus its dependent claims) includes subcutaneously administering a bolus of a drug to a patient, which constitutes a method of treatment of the human body by therapy, practised on the human body.

## ANNEX TO THE EUROPEAN SEARCH REPORT
## ON EUROPEAN PATENT APPLICATION NO.

EP 08 00 3164

This annex lists the patent family members relating to the patent documents cited in the above-mentioned European search report.
The members are as contained in the European Patent Office EDP file on
The European Patent Office is in no way liable for these particulars which are merely given for the purpose of information.

04-07-2008

| Patent document cited in search report | Publication date | Patent family member(s) | Publication date |
|---|---|---|---|
| US 2003160683 A1 | 28-08-2003 | US 2006132292 A1<br>US 2005030164 A1 | 22-06-2006<br>10-02-2005 |
| WO 2006124716 A | 23-11-2006 | CA 2612714 A1<br>EP 1881786 A2 | 23-11-2006<br>30-01-2008 |
| US 2003114836 A1 | 19-06-2003 | AT 344070 T<br>AU 2002366794 A1<br>CA 2471007 A1<br>DE 60215856 T2<br>DK 1458435 T3<br>EP 1458435 A2<br>JP 2005512689 T<br>WO 03053498 A2<br>US 2005245904 A1<br>US 2007287985 A1 | 15-11-2006<br>09-07-2003<br>03-07-2003<br>06-06-2007<br>19-03-2007<br>22-09-2004<br>12-05-2005<br>03-07-2003<br>03-11-2005<br>13-12-2007 |
| WO 2006089965 A | 31-08-2006 | CN 101128229 A<br>EP 1866010 A1<br>US 2008147041 A1 | 20-02-2008<br>19-12-2007<br>19-06-2008 |
| EP 1338295 A | 27-08-2003 | AU 2003200685 A1<br>AU 2003213593 A1<br>CA 2420136 A1<br>CN 1449840 A<br>JP 2003290345 A<br>MX PA03001781 A<br>NO 20030850 A<br>PL 358897 A1<br>WO 03071930 A2 | 11-09-2003<br>09-09-2003<br>26-08-2003<br>22-10-2003<br>14-10-2003<br>16-08-2005<br>27-08-2003<br>08-09-2003<br>04-09-2003 |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6878132 B
- EP 0991440 A
- US 6248093 B
- US 20050238507 A
- US 6736796 B

- US 4562751 A
- WO 2007056592 A
- EP 1124600 B1
- EP 0991440 B1